# EUROPÄISCHE PATENTSCHRIFT

(11) **EP 0 951 895 B1**
(45) Veröffentlichungstag und Bekanntmachung des Hinweises auf die Patenterteilung: **22.11.2006**
(21) Anmeldenummer: 99106169.8
(22) Anmeldetag: 08.04.1999
(51) Int. Cl.: A61K 6/08, A61K 6/00

(54) **Zusammensetzung zum Füllen von Zahnwurzelkanälen**
Composition for filling root canals of teeth
Composition pour l'obturation des canaux dentaires

(30) Priorität: 22.04.1998 DE 19817844
(43) Veröffentlichungstag der Anmeldung: 27.10.1999
(73) Patentinhaber: Coltène/Whaledent GmbH + Co. KG, 89129 Langenau (DE)
(72) Erfinder: Mannschedel, Werner, 89129 Langenau (DE)
(74) Vertreter: Forstmeyer, Dietmar

(56) Entgegenhaltungen:
- WO-A-99/56701
- DE-A- 19 501 374
- DE-A- 19 709 531
- US-A- 4 632 977
- US-A- 4 657 592
- US-A- 4 931 096
- DATABASE BIOSIS [Online] BIOSCIENCES INFORMATION SERVICE, PHILADELPHIA, PA, US; 1995 SJOGREN ULF ET AL: "Tissue reaction to gutta-percha particles of various sizes when implanted subcutaneously in guinea pigs" Database accession no. PREV199698560017 XP002259832 & EUROPEAN JOURNAL OF ORAL SCIENCES, Bd. 103, Nr. 5, 1995, Seiten 313-321, ISSN: 0909-8836

## Beschreibung

Die vorliegende Erfindung betrifft eine Zusammensetzung zum Füllen von Zahnwurzelkanälen, die ein Pulver auf Isoprenbasis und mindestens einen Sealer umfaßt.

Zur Therapie einer Erkrankung, die als Pulpitis bekannt ist, wird die erkrankte Pulpa mechanisch aus dem Wurzelkanal entfernt, der Wurzelkanal gereinigt und ausgebohrt, mit einem elastisch-plastischen Element oder einem anderen Füllmaterial gefüllt und danach versiegelt. Ein ideales Wurzelkanalfüllmittel sollte das periapikale Gewebe nicht reizen, die Wurzelkanäle lateral und vertikal dicht verschließen, volumenbeständig sein und im Wurzelkanal nicht schrumpfen. Zum Stand der Technik sei beispielsweise auf Friedman et al. in J. Dent. Res., 54 (1975) 921-925, Briseno in Philipp J., 2, 90, 65-73 und die US-A-4 632 977 verwiesen. Briseno beschreibt als Wurzelkanalfüllmaterialien unter anderem halbfeste Zemente auf Kunstharzbasis, Zinkoxid-Eugenol-Basis, Calciumhydroxidbasis und Glas-Ionomerbasis. Die US-A-4 632 977 schlägt Füllmaterialien auf Trans-Poly-Isopren-Basis vor, beispielsweise auf Basis von Guttapercha oder Balata. DE-A-195 01 374 offenbart ein elastisch-plastisches Element mit einer Matrix auf Isoprenbasis und mit mindestens einem Füllstoff, wobei das Element zum Füllen von Wurzelkanälen des Menschen oder eines Tieres geeignet ist. Das elastisch-plastische Element kann in Form einer Guttapercha-Spitze vorliegen. Im Handel sind Guttapercha-Spitzen erhältlich, deren Standardzusammensetzung 20 Gew.-% Guttapercha als Matrix, 60 bis 75 Gew.-% Zinkoxid als Füllstoff, 1 bis 17 Gew.-% Schwermetallsulfate als Röntgenkontrastmittel und 3 bis 4 Gew.-% Wachse und Harze als Weichmacher vorsieht. Dieses bekannte Füllmaterial ist im Wurzelkanal inert und reagiert demgemäß nicht mit dem Körpergewebe.

Derartige Guttaperchastifte werden üblicherweise durch die sogenannte vertikale oder laterale Kondensation in den Wurzelkanal eingebracht:

Bei der vertikalen Kondensation wird ein Guttaperchastift in den Wurzelkanal eingeführt und seine Lage im Kanal mittels Röntgenbild kontrolliert. Dann wird Sealer zusammen mit der Guttapercha in den Kanal eingebracht und der Wurzelkanal durch abwechselndes Einführen und Kondensieren der thermoplastischen Guttapercha in mehreren Arbeitsgängen vollständig gefüllt. Weiter kann erhitzte und erweichte Guttapercha auf einem Trägerstift in den Wurzelkanal eingeführt werden. Schließlich kann auch geschmolzene Guttapercha mit einer Guttaperchapistole eingefüllt werden.

Dieses Verfahren weist jedoch den Nachteil auf, daß die Hitze das um den Zahn herumliegende Gewebe denaturieren und somit schädigen kann. Ferner kann das Guttapercha beim Erwärmen oxydiert und/oder abgebaut bzw. gecrackt werden. Insbesondere wenn Guttapercha wiederholt erhitzt wird kann es zu einem Abbau und damit zur Alterung des Materials führen kommen.

Bei der lateralen Kondensation werden Guttaperchastifte nacheinander in einen Wurzelkanal eingebracht und gegen die Seiten des Wurzelkanals gepreßt bis der Wurzelkanal vollständig gefüllt ist. Ein Nachteil dieses Verfahrens besteht darin, daß die Guttaperchastifte mit einem gewissen Kraftaufwand in den Wurzelkanal gepreßt werden müssen, wodurch es zur Fraktur der Zähne kommen kann. Zusätzlich besteht bei der vertikalen Kondensation die Gefahr des Überpressens wodurch Guttaperchastifte in das periapikale Gewebe geschoben werden können, was dort zu Reizungen oder sogar zu Entzündungen führen kann.

Ferner ist auch die Filmdicke des Sealers von Bedeutung. Diese kann bei der lateralen Kondensation jedoch nicht genau eingestellt werden.

Erfindungsgemäß wird daher eine Zusammensetzung zum Füllen von Zahnwurzelkanälen bereitgestellt, die ein Pulver auf Isoprenbasis und mindestens einen Sealer umfaßt.

Vorzugsweise ist das Pulver auf Isoprenbasis Trans-Poly-Isopren, Guttapercha, Balata oder ein Gemisch davon, wobei Guttaperchapulver bevorzugt wird. In der Beschreibung der vorliegenden Erfindung soll der Ausdruck Guttapercha auch Isopren-, Trans-Poly-Isopren oder Balata umfassen. Bevorzugt bezeichnet er aber nur Guttapercha selbst. Bei dem Guttapercha handelt es sich vorzugsweise um Transpolyisopren.

Erfindungsgemäß ist es auch möglich, Zusammensetzung mit verschiedenen Guttapercha : Sealer-Gewichtsverhältnissen von 50 : 50 bis 10 : 90 zu verwenden: So kann z.B. als unterste Schicht eine Zusammensetzung mit einem Guttapercha : Sealer-Verhältnis von 50 : 50 und darauf eine Zusammensetzung eingesetzt werden, die ein anderes Guttapercha : Sealer-Verhältnis von z.B. 30 : 70 aufweist.

Durch einen derartigen Schichtenaufbau wird eine optimale biologische Kompatibilität mit einem für die Haftung am Zahn und den Zusammenhalt der Masse idealen Guttapercha : Sealer-Verhältnis vereint. Außerdem wird eine gleichmäßige Bedeckung des Wurzelkanals mit Sealer möglich, so daß eine gute Haftung des Füllmaterials am Zahn gewährleistet wird, zumal mit der erfindungsgemäßen Zusammensetzung eine homogenere Füllung möglich ist, als mit Guttaperchaspitzen, da die Zwischenräume zwischen den Pulverteilchen geringer sind.

Gemäß einer bevorzugten Ausführungsform weist das Pulver auf Isoprenbasis erfindungsgemäß einen Gehalt von mindestens 80 Gew.-% Trans-Poly-Isopren auf.

Erfindungsgemäß kann ein Wurzelkanal auch mit der erfindungsgemäßen Zusammensetzung und einem oder mehreren an sich üblichen Guttaperchastiften gefüllt werden.

Das Pulver auf Isoprenbasis weist erfindungsgemäß vorzugsweise eine Korngröße von bis zu 100 µm, stärker bevorzugt von bis zu 50 µm, noch stärker bevorzugt von 5 bis 30 µm und besonders bevorzugt von 10 bis zu 20 µm auf. Erfindungsgemäß kann das Guttaperchapulver zum Beispiel eine Korngröße von 15 bis 20 µm aufweisen. Erfindungsgemäß können aber auch kleinere Korngrößen eingesetzt werden. Je nach Teilchengröße kann das Material auf Isoprenbasis auch als Granulat oder Grieß vorliegen.

Weist das Pulver eine Korngröße von mehr als 100 µm auf, so paßt es nicht mehr in feine Nebenkanäle der Wurzelkanäle; eine Korngröße von weniger als 5 µm ist zur Zeit technisch allerdings nur schwer realisierbar.

Bevorzugt werden die in Kombination mit dem Guttaperchapulver eingesetzten Sealer so ausgewählt, daß sie in Wasser unlöslich sind, da es sonst zu einer Resorption des Sealers kommen kann, was unter physiologischen Gesichtspunkten unerwünscht ist. Außerdem bewirkt ein Auswaschen des Sealers Läsionen im Füllmaterial, was zu einem erneuten Bakterienbefall des Wurzelkanals führen kann. Damit das Guttaperchapulver in dem Füllmaterial fest bleibt wird bevorzugt, daß es in dem oder den eingesetzten Sealer(n) unlöslich ist.

Als Sealer können übliche Sealer verwendet werden, die Kunstharze wie Epoxybisphenole, Eugenol, Silikone etc. enthalten. Derartige Sealer umfassen zum Beispiel handelsübliche Silikonsealer vom Ein- oder Zwei-Komponententyp, wobei eine der beiden Komponenten einen Katalysator, wie eine Platinverbindung, enthalten kann. Besonders werden die Sealer bevorzugt, die in der DE 197 09 531.3 offenbart sind.

Die erfindungsgemäße Zusammensetzung weist außerdem den Vorteil auf, daß das biologisch unbedenkliche Guttapercha, welches bisher nur mühsam und unter Einsatz einer Vielzahl von Arbeitsschritten eingesetzt wurde, in einer das Einbringen in die Wurzelkanäle erheblich vereinfachenden Form bereitgestellt wird.

Erfindungsgemäß enthält die Gesamtzusammensetzung vorzugsweise mindestens 1 Gew.-% Trans-Poly-Isopren und bis zu 99 Gew.-% Sealer. Vorzugsweise werden mindestens 20 Gew.-%, stärker bevorzugt mindestens 30 Gew.-%, noch stärker bevorzugt mindestens 40 Gew.-%, noch stärker bevorzugt mindestens 50 Gew.-%, und am stärksten bevorzugt so viel Sealer eingesetzt, daß das gewählte Guttaperchapulver in der gewünschten Korngröße in der gegebenenfalls gewählten Schicht im Wurzelkanal optimale physiologische und Hafteigenschaften aufweist. Die entsprechenden Verhältnisse können von Fachleuten durch einige Versuche herausgefunden werden.

Die erfindungsgemäße Zusammensetzung kann zusätzlich übliche Zusatzstoffe, wie Füllstoffe, pharmazeutische Wirkstoffe, Röntgenkontrastmittel, Farbstoffe wie Eisenoxide, Wachse, Tenside, Fettsäuren wie Stearinsäure, Antioxidantien, Titandioxid und Magnesiumoxid oder Gemische davon enthalten.

Als Füllstoffe können an sich übliche Stoffe wie Zinkoxid und/oder Calciumhydroxid in an sich üblichen Mengen eingesetzt werden.

Als pharmazeutische Wirkstoffe kommen insbesondere in wäßrigem Medium lösbare oder dispergierbare Wirkstoffe, beispielsweise ein Antibiotikum und/oder Glukokortikoid in Betracht. Eine erfindungsgemäße Zusammensetzung kann vorzugsweise durch
(a) bis zu 50 Gew.-% Guttaperchapulver,
(b) bis zu 99 Gew.-% Sealer,
(c) bis zu 70 Gew.-% Füllstoff,
(d) bis zu 70 Gew.-% Röntgenkontrastmittel, bezogen auf (a) und (b), und
(e) gegebenenfalls zusätzliche übliche Komponenten gekennzeichnet sein.

So kann die erfindungsgemäße Zusammensetzung beispielsweise durch
(a) bis zu 50 Gew.-% Guttaperchapulver,
(b) bis zu 50 Gew.-% Sealer,
(c) bis zu 50 Gew.-% Zinkoxid,
(d) bis zu 50 Gew.-% Röntgenkontrastmittel, bezogen auf (a) und (b), und
(e) gegebenenfalls zusätzliche übliche Komponenten gekennzeichnet sein.

Gemäß einer weiteren Ausführungsform der Erfindung wird schließlich ein Verfahren zur Herstellung der erfindungsgemäßen Zusammensetzung vorgesehen, das dadurch gekennzeichnet ist, daß man ein Material auf Isoprenbasis zerkleinert und gegebenenfalls vor, während oder nach der Zerkleinerung mit Zusatzstoffen versetzt. Dann kann das Pulver mit einem oder mehreren Sealern oder deren Komponenten gemischt werden.

Zur Zerkleinerung von Guttapercha können verschiedene Verfahren eingesetzt werden, zum Beispiel Versprühen von geschmolzener oder gelöster Guttapercha in einem Sprühturm, Polymerisation in einem Nicht-Lösungsmittel, übliche Fällungsverfahren und bevorzugt Mahlen bei tiefen Temperaturen. Dazu wird Guttapercha zum Beispiel mit flüssigem Stickstoff auf tiefe Temperaturen von bis zu -270 °C abgekühlt und dann gemahlen, bis der gewünschte Teilchendurchmesser, zum Beispiel 15 bis 20 um erreicht wird.

Ein derartiges Guttaperchapulver kann leicht in Kombination mit einem viskosen Sealer mit einer Spritze appliziert werden, dringt gut in feine Nebenkanälchen ein und liegt gut an den Zahnwänden an. Ein Erhitzen oder ein kräftiges Nachstopfen mit einem Plugger kann unterbleiben.

Die erfindungsgemäßen Zusammensetzungen können neben dem Pulver auf Isoprenbasis und dem Sealer weitere organische und anorganische Stoffe enthalten, die - vollständig oder in mehreren Portionen - vor, während oder nach dem Granulieren oder Mahlen zugesetzt werden.
Die erfindungsgemäße Zusammensetzung weist aber im wesentlichen drei Komponenten auf, die im folgenden noch näher erläutert werden sollen.

### Komponente 1

Die mechanischen Eigenschaften des erfindungsgemäß herstellbaren Pulvers werden in erster Linie durch die Eigenschaften des Materials auf Isoprenbasis bestimmt. Das Pulver sollte andere Komponenten, insbesondere einen Füllstoff und gegebenenfalls ein Röntgenkontrastmittel leicht aufnehmen können. Für diese Zwecke hat sich eine Matrix mit mindestens 80 Gew.-% Trans-Poly-Isopen als vorteilhaft erwiesen. Als Beispiel kann Guttapercha angeführt werden, bei dem es sich um eine Matrix auf natürlicher Basis handelt, deren Hauptbestandteil Trans-Poly-Isopren ist. Selbstverständlich sind auch andere Trans-Poly-Isoprene einsetzbar, wie Balata, synthetische Matrizen auf Isopren-Basis oder Abkömmlinge der genannten Materialien.

### Komponente 2

Die erfindungsgemäße Zusammensetzung kann auch Füllstoffe wie Zinkoxid und/oder Calciumhydroxid enthalten. Diese Füllstoffe liegen, bezogen auf die gesamte Zusammensetzung, beispielsweise in einer Menge von bis zu 80 Gew.-% neben bis zu 50 Gew.-% Material auf Isoprenbasis vor. Bei Calciumhydroxid handelt es sich um einen gewebe- bzw. körperverträglichen Stoff, der mit der Matrix gemischt und zusätzlich mit pharmazeutischen Wirkstoffen sowie üblichen Hilfsmitteln compoundiert werden kann. Zinkoxid läßt sich als Pulver in Guttapercha einwalzen, was den Herstellungsprozeß recht einfach gestalten läßt. Der Anteil an Zinkoxid orientiert sich dann an der Menge des Guttaperchapulvers und dessen Aufnahmevermögen für einen Füllstoff. Ein Beispiel für eine Zusammensetzung mit einem recht hohen Füllstoff-Anteil ist ein Guttaperchapulver mit einem Gehalt von etwa 70 Gew.-% Zinkoxid neben etwa 30 Gew.-% Guttapercha.

### Komponente 3

Als weitere, allerdings fakultative, wenn auch übliche Komponente kann ein Röntgenkontrastmittel aus der Gruppe der Zink-, Ytterbium-, Yttrium-, Gadolinium-, Zirkonium-, Strontium-, Wolfram-, Tantal-, Niob-, Barium-, Wismut-, Molybdän- und Lanthanpulver, pulvrigen Legierungen davon, Oxiden, Fluoriden, Sulfaten, Carbonaten, Wolframaten und Carbiden davon, und Gemischen davon ausgewählt werden. Diese Komponente kann in einer Menge von bis zu 70 Gew.-%, vorzugsweise bis zu 50 Gew.-%, stärker bevorzugt bis zu 30 Gew.-%, bezogen auf die Zusammensetzung, vorgesehen werden.

### Beispiel

Zur Herstellung von Guttaperchapulver wurde Guttapercha als Matrix zunächst zwischen einer Trägerwalze und einer Andruckwalze verwalzt, so daß sich die Guttapercha als dünner Film bzw. fellartig um die Trägerwalze legte. Danach wurden pulverförmiges Zinkoxid und pulverförmiges Zirkonoxid in einem Gewichtsverhältnis von 30 % Guttapercha : 55 % Zinkoxid : 15 % Zirkonoxid in den gebildeten Film eingewalzt, der Film von der Trägerwalze abgeschält, der abgeschälte Film zerkleinert und zu einem Strang extrudiert. Dieser Strang wurde danach zerschnitten und zerkleinert, mit flüssigem Stickstoff auf eine Temperatur von -270 °C abgekühlt und dann bis zu einem Teilchendurchmesser von etwa 20 µm gemahlen. Jeweils 2 g des so erhaltenen Pulvers wurden dann zu jeweils einer Komponente eines Zweikomponentensealers gegeben. In jeder Komponente lagen Pulver : Sealer in einem Gewichtsverhältnis von 40 : 60 vor. Unmittelbar vor der Verwendung werden die beiden Komponenten vereint und gemischt.

Das Produkt läßt sich leicht in einen Kanal einbringen, ermöglicht außerdem ein Einführen eines Guttaperchastiftes und dichtet den Kanal gut ab.

## Patentansprüche

1. Zusammensetzung zum Füllen von Zahnwurzelkanälen, die ein Pulver auf Isoprenbasis und mindestens einen Sealer umfaßt.

2. Zusammensetzung nach Anspruch 1, **dadurch *gekennzeichnet*, daß** das Pulver auf Isoprenbasis ein Trans-Poly-Isopren-, Guttapercha- oder Balatapulver oder ein Gemisch davon ist.

3. Zusammensetzung nach Anspruch 1 oder 2, **dadurch *gekennzeichnet*, daß** das Pulver auf Isoprenbasis mindestens 80 Gew.-% Trans-Poly-Isopren umfaßt.

4. Zusammensetzung nach einem der vorhergehenden Ansprüche, **dadurch *gekennzeichnet*, daß** das Pulver eine Korngröße von bis zu 100 µm, bevorzugt von 10 bis 30 µm aufweist.

5. Zusammensetzung nach einem der vorhergehenden Ansprüche, **dadurch *gekennzeichnet*, daß** der Sealer in Wasser unlöslich ist.

6. Zusammensetzung nach einem der vorhergehenden Ansprüche, **dadurch *gekennzeichnet,* daß** sie bis zu 50 Gew.-% Trans-Poly-Isoprenpulver und bis zu 99 Gew.-% Sealer enthält.

7. Zusammensetzung nach einem der vorhergehenden Ansprüche, **dadurch *gekennzeichnet*, daß** das Pulver auf Isoprenbasis übliche Zusatzstoffe, ausgewählt aus Füllstoffen, pharmazeutischen Wirkstoffen, Röntgenkontrastmitteln, Füllstoffen, Wachsen, Harzen, Tensiden oder Gemische davon enthält.

8. Zusammensetzung nach Anspruch 7, **dadurch *gekennzeichnet*, daß** der Füllstoff Calciumhydroxid, Zinkoxid, Aluminiumoxid, Siliciumdioxid oder Gemische davon umfaßt.

9. Zusammensetzung nach einem der Ansprüche 7 oder 8, **dadurch *gekennzeichnet*, daß** der pharmazeutische Wirkstoff ein in wäßrigen Medien löslicher oder dispergierbarer Wirkstoff ist.

10. Zusammensetzung nach Anspruch 9, **dadurch *gekennzeichnet*, daß** der Wirkstoff ein antibakteriell wirksames Mittel wie ein Antibiotikum und/oder ein Glucocorticoid ist.

11. Zusammensetzung nach einem der Ansprüche 7 bis 9, **dadurch *gekennzeichnet*, daß** das Röntgenkontrastmittel aus der Gruppe der Zink-, Ytterbium-, Yttrium-, Gadolinium-, Zirkonium-, Strontium-, Wolfram-, Tantal-, Niob-, Barium-, Wismut-, Molybdän- und Lanthanpulver, pulvrigen Legierungen davon, Oxiden, Fluoriden, Sulfaten, Carbonaten, Wolframaten und Carbiden davon, und Gemischen davon ausgewählt wird.

12. Zusammensetzung nach einem der vorhergehenden Ansprüche, **dadurch *gekennzeichnet*, daß** alle Komponenten außer dem Sealer als Pulver oder als viskose Masse vorliegen.

13. Zusammensetzung nach einem der vorhergehenden Ansprüche, ***gekennzeichnet* durch**
(a) bis zu 50 Gew.-% Guttaperchapulver,
(b) bis zu 99 Gew.-% Sealer,
(c) bis zu 70 Gew.-% Füllstoff,
(d) bis zu 70 Gew.-% Röntgenkontrastmittel, bezogen auf (a) und (b), und
(e) gegebenenfalls zusätzliche übliche Komponenten.

14. Zusammensetzung nach Anspruch 13, ***gekennzeichnet* durch**
(a) bis zu 50 Gew.-% Guttaperchapulver,
(b) bis zu 50 Gew.-% Sealer,
(c) bis zu 50 Gew.-% Zinkoxid,
(d) bis zu 50 Gew.-% Röntgenkontrastmittel, bezogen auf (a) und (b), und
(e) gegebenenfalls zusätzliche übliche Komponenten.

15. Verfahren zur Herstellung einer Zusammensetzung nach einem der vorstehenden Ansprüche, **dadurch *gekennzeichnet*, daß** das Material auf Isoprenbasis zerkleinert, vor, während oder nach der Zerkleinerung mit den gegebenenfalls vorhandenen Zusatzstoffen versetzt und dann mit mindestens einem Sealer gemischt wird.

16. Verfahren nach Anspruch 15, **dadurch *gekennzeichnet*, daß** das Material auf Isoprenbasis auf mindestens -40 °C, stärker bevorzugt auf mindestens -200 °C und am stärksten bevorzugt auf -270 °C abgekühlt und dann zerkleinert wird.

17. Verfahren nach Anspruch 15 oder 16, **dadurch *gekennzeichnet*, daß** das Material auf Isoprenbasis nach der Abkühlung auf eine Korngröße nach Anspruch 4 gemahlen wird.

## Claims

1. Composition for filling tooth root canals comprising an isoprene-based powder and at least one sealer.

2. Composition according to claim 1, **characterised in that** the isoprene-based powder is a trans-polyisoprene, gutta-percha or balata powder or a mixture thereof.

3. Composition according to claim 1 or 2, **characterised in that** the isoprene-based powder comprises at least 80% by weight of trans-polyisoprene.

4. Composition according to any one of the preceding claims, **characterised in that** the powder has a particle size of up to 100 µm, preferably from 10 to 30 µm.

5. Composition according to any one of the preceding claims, **characterised in that** the sealer is insoluble in water.

6. Composition according to any one of the preceding claims, **characterised in that** it comprises up to 50% by weight of trans-polyisoprene powder and up to 99% by weight of sealer.

7. Composition according to any one of the preceding claims, **characterised in that** the isoprene-based powder comprises customary additives, selected from fillers, pharmaceutical active ingredients, X-ray contrast agents, fillers, waxes, resins, surfactants or mixtures thereof.

8. Composition according to claim 7, **characterised in that** the filler comprises calcium hydroxide, zinc oxide, aluminium oxide, silicon dioxide or mixtures thereof.

9. Composition according to either claim 7 or 8, **characterised in that** the pharmaceutical active ingredient is an active ingredient that is soluble or dispersible in aqueous media.

10. Composition according to claim 9, **characterised in that** the active ingredient is an anti-bacterially active composition, such as an antibiotic and/or a glucocorticoid.

11. Composition according to any one of claims 7 to 9, **characterised in that** the X-ray contrast agent is selected from the group consisting of zinc, ytterbium, yttrium, gadolinium, zirconium, strontium, tungsten, tantalum, niobium, barium, bismuth, molybdenum and lanthanum powders, powdered alloys thereof, oxides, fluorides, sulphates, carbonates, tungstates and carbides thereof, and mixtures thereof.

12. Composition according to any one of the preceding claims, **characterised in that** all the components except the sealer are present in the form of a powder or a viscous mass.

13. Composition according to any one of the preceding claims,
**characterised by**
(a) up to 50% by weight of gutta-percha powder,
(b) up to 99% by weight of sealer,
(c) up to 70% by weight of filler,
(d) up to 70% by weight of X-ray contrast agent, based on (a) and (b), and
(e) optionally additional customary components.

14. Composition according to claim 13, **characterised by**
(a) up to 50% by weight of gutta-percha powder,
(b) up to 50% by weight of sealer,
(c) up to 50% by weight of zinc oxide,
(d) up to 50% by weight of X-ray contrast agent, based on (a) and (b), and
(e) optionally additional customary components.

15. Method for the preparation of a composition according to any one of the preceding claims, **characterised in that** the isoprene-based material is comminuted, the additives which may be present are added before, during or after comminution, and the mixture is then mixed with at least one sealer.

16. Method according to claim 15, **characterised in that** the isoprene-based material is cooled to at least -40°C, especially to at least -200°C and more especially to -270°C, and is then comminuted.

17. Method according to claim 15 or 16, **characterised in that** the isoprene-based material is ground to a particle size according to claim 4 after cooling.

## Revendications

1. Composition pour l'obturation des canaux dentaires, comprenant une poudre à base d'isoprène et au moins, un agent de scellement.

2. Composition selon la revendication 1, **caractérisée en ce que** la poudre à base d'isoprène est une poudre de trans-poly-isoprène, de gutta-percha ou de balata ou un mélange de celles-ci.

3. Composition selon la revendication 1 ou 2, **caractérisée en ce que** la poudre à base d'isoprène comprend au moins 80 % en poids de trans-poly-isoprène.

4. Composition selon l'une quelconque des revendications précédentes, **caractérisée en ce que** la poudre présente une taille de particules allant jusqu'à 100 µm, de préférence de 10 à 30 µm.

5. Composition selon l'une quelconque des revendications précédentes, **caractérisée en ce que** l'agent de scellement est insoluble dans l'eau.

6. Composition selon l'une quelconque des revendications précédentes, **caractérisée en ce qu'**elle contient jusqu'à 50 % en poids de poudre de trans-poly-isoprène et jusqu'à 99 % en poids d'agent de scellement.

7. Composition selon l'une quelconque des revendications précédentes, **caractérisée en ce que** la poudre à base d'isoprène contient des adjuvants habituels choisis parmi les substances de charge, les principes actifs pharmaceutiques, les agents de contraste radiographique, les charges, les cires, les résines, les tensioactifs ou leurs mélanges.

8. Composition selon la revendication 7, **caractérisée en ce que** la substance de charge comprend de l'hydroxyde de calcium, de l'oxyde de zinc, de l'oxyde d'aluminium, de l'oxyde de silicium ou leurs mélanges.

9. Composition selon la revendication 7 ou 8, **caractérisée en ce que** le principe actif pharmaceutique est un principe actif soluble ou dispersible dans un milieu aqueux.

10. Composition selon la revendication 9, **caractérisée en ce que** le principe actif est un agent efficace d'un point de vue anti-bactérien, tel qu'un antibiotique et/ou un glucocorticoïde.

11. Composition selon l'un quelconque des revendications 7 à 9, **caractérisée en ce que** l'agent de contraste radiographique est choisi dans le groupe des poudres de zinc, d'ytterbium, d'yttrium, de gadolinium, de zirconium, de strontium, de wolfram, de tantale, de niobium, de baryum, de bismuth, de molybdène et de lanthane, leurs alliages pulvérulents, des oxydes, fluorures, sulfates, carbonates, wolframates et carbures et leurs mélanges.

12. Composition selon l'une quelconque des revendications précédentes, **caractérisée en ce que** tous les composants à l'exception de l'agent de scellement se présentent sous forme de poudre ou de masse visqueuse.

13. Composition selon l'une quelconque des revendications précédentes, **caractérisée par**
(a) jusqu'à 50 % en poids de poudre de gutta-percha,
(b) jusqu'à 99 % en poids d'agent de scellement,
(c) jusqu'à 70 % en poids de substance de charge,
(d) jusqu'à 70 % en poids d'agent de contraste radiographique, par rapport aux éléments (a) et (b), et
(e) éventuellement, d'autres composants habituels.

14. Composition selon la revendication 13, **caractérisée par**
(a) jusqu'à 50 % en poids de poudre de gutta-percha,
(b) jusqu'à 50 % en poids d'agent de scellement,
(c) jusqu'à 50 % en poids d'oxyde de zinc,
(d) jusqu'à 50 % en poids d'agent de contraste radiographique, par rapport aux éléments (a) et (b), et
(e) éventuellement, d'autres composants habituels.

15. Procédé de production d'une composition selon l'une quelconque des revendications précédentes, **caractérisé en ce que** le matériau à base d'isoprène est réduit, est ajouté avant, pendant ou après réduction, aux adjuvants éventuellement présents et est ensuite mélangé avec au moins un agent de scellement.

16. Procédé selon la revendication 15, **caractérisé en ce que** le matériau à base d'isoprène est refroidi à au moins -40° C, de manière davantage préférée, à -200° C et de manière préférée entre toutes, à -270° C et est ensuite réduit.

17. Procédé selon la revendication 15 ou 16, **caractérisé en ce que** le matériau à base d'isoprène est broyé après refroidissement, à une taille de particules selon la revendication 4.
